# EUROPEAN PATENT APPLICATION

(11) **EP 2 463 361 A1**
(43) Date of publication of application: **13.06.2012**
(21) Application number: 10194486.6
(22) Date of filing: 10.12.2010
(51) Int. Cl.: C12N 5/0735

(54) **Methods to derive stem cells and to induce the proliferation of a single isolated blastomere**

(71) Applicant: Universitat Autònoma De Barcelona, 08193 Bellaterra (Barcelona) (ES)
(72) Inventor: Santalo Pedro, Josep, 08193 Bellaterra (ES); Ibanez de Sans, Elena, 08193 Bellaterra (ES); Gonzalez Garrido, Sheyla, 08291 Ripollet (Barcelona) (ES)
(74) Representative: ZBM Patents

(57) **Abstract**

These in vitro methods comprise the step of incubating an isolated blastomere obtained from an embryo before compaction with a molecule capable of stimulating the activity of the endogenous E-cadherin during the appropriate time to induce the division of the blastomere but not the differentiation of the blastomere. The method for deriving embryonic stem cells comprises the additional step of incubating the blastomeres resulting from step (a) in an embryonic stem cell medium.

## Description

The present invention relates to novel methods for deriving embryonic (ESC) and method for stimulating the division of a single isolated blastomere.

### BACKGROUND ART

Embryonic stem cells (ESC) are traditionally derived from the inner cell mass (ICM) of blastocysts. There are several drawbacks to this technique, namely its low efficiency and also the fact that this derivation procedure destroys the blastocyst-stage embryo. Mainly ethical issues associated with the destruction of embryos during this process have led to search for alternative methods.

The alternative to the use of CM of blastocysts to derive ESC is the generation of ESC from an isolated blastomere. This technique overcomes the above main drawback, namely, the destruction of the embryo. The derivation of ESC from isolated blastomeres at different developmental stages revealed very low establishment rates (i.e. blastomere division and derivation rates) when an isolated blastomere from an eight-cell stage embryo (1/8) was used.

Different attempts have been made in order to improve the efficiency of deriving ESC from isolated blastomeres. In this regard, several compounds have been used in the state of the art to supplement the blastomere medium, in order to improve the low efficiency of the currently stem cell (SC) derivation available techniques from an isolated blastomere. Typically, as disclosed in Chung *et al.* (2008) and Klimanskaya *et al.* (2006), laminin and fibronectin have been used in the medium to grow a blastomere extracted from an eight-cell stage embryo. However, the efficiency rates achieved using those compounds are not as high as expected; and furthermore, given the sample size of the replicates (n=2), the results can not be considered as statistically significant.

Therefore, there is the need of further methods that allow the efficient division and derivation of SC from an isolated blastomere.

### SUMMARY OF THE INVENTION

The inventors of the present application have found that when exogenous E-cadherin protein is used, an efficient division of a single isolated blastomere obtained from an embryo. This means that starting from an isolated blastomere; a high number of blastomeres are obtained due to the division stimulation effect of the E-cadherin and the maintenance of the undifferentiated state of the cell induced by E-cadherin. Both effects have positive repercussions on the subsequent steps of derivation of stem cells, either embryonic or trophoblast, because a high number of this type of cells from a single isolated blastomere is obtained.

The effects observed with the E-cadherin can be achieved with any other known substance which stimulates the activity of the endogenous E-cadherin of the isolated blastomere.

The state of the art provides no teaching regarding the role of E-cadherin, and molecules capable of stimulating the activity of endogenous E-cadherin, in the proliferation of blastomeres. The only teaching regarding the possible applications of E-cadherin and molecules capable of stimulating the activity of endogenous E-cadherin, in the field of SC, are limited to the use of E-cadherin in culture mouse ESC to increase their proliferative ability (Nagaoka *et al.* 2006); and, contradictorily, the use of antibodies against E-cadherin, i.e. abrogation of the E-cadherin, to obtain a single murine ESC (mESC) suspension culture to allow the expansion of said undifferentiated mESC over prolonged periods (Mohamet *et al.* 2010). The contradictory disclosures of Nagaoka *et al.* and Mohamet *et al.* show the limited knowledge of the role of E-cadherin in the field of ESC.

In contrast to the teachings of Ngaoka *et al.* and Mohamet *et al.* linking the E-cadherin and the abrogation of E-cadherin, respectively, only with ESC proliferative ability, the present invention is based on the unexpected inventors' finding that an early treatment of blastomeres with an approach that resembles native signalling of E-cadherin-mediated junctions has an effect on the efficiency of blastomere division and subsequent SC derivation processes.

The findings of the present inventors are also not anticipated by the known role of fibronectin and laminin in a growing medium for an isolated blastomeres, as discussed above, given the completely different structures and biological roles of the E-cadherin, laminin and fibronectin, i.e. laminin and fibronectin are molecules present on the extracellular matrix; whereas the E-cadherin is a cell surface protein.

Thus, the present invention provides in one aspect an in vitro method for inducing the proliferation of an isolated blastomere obtained from an embryo before compaction, comprising the step of incubating the isolated blastomere, with a molecule capable of stimulating the activity of the endogenous E-cadherin during the appropriate time to induce the division of the blastomere but not the differentiation of the blastomere.

In a further aspect, the present invention provides in a first aspect an in vitro method for deriving embryonic stem cells, the method comprising the steps of:
(a) incubating an isolated blastomere, obtained from an embryo before compaction, with a molecule capable of stimulating the activity of the endogenous E-cadherin during the appropriate time to induce the division of the blastomere but not the differentiation of the blastomere; and (b) incubating the blastomeres resulting from step (a) in an embryonic stem cell medium
The method for deriving ESC of the present invention is characterised by a high derivation rate. The resulting ESC maintain their pluripotency for more than 7 passages, and are also capable to differentiate into all three germ layers.

In still a further aspect, the present invention provides an in vitro method for deriving trophoblast stem cells comprising the step of incubating an isolated blastomere obtained from an embryo before compaction, in a trophoblast stem cell medium and in the presence of a molecule capable of stimulating the activity of the endogenous E-cadherin, during the appropriate time to induce the differentiation of the trophoblast.

### DETAILED DESCRIPTION OF THE INVENTION

For the sake of clarity a definition of expressions used in the present invention are provided:
The expression "embryo before compaction" is to be understood as an embryo with 2, 4 or 8 blastomeres.
The expression "8 cell stage embryo" refers to an embryo consisting of 8 cells.
The expression "1/8 blastomere" refers to the blastomere isolated from an 8 cell stage embryo.
The expression "blastomere differentiation" is to be understood as the commitment of a blastomere towards cellular defined fate, in other words, the process by which a less specialised cell becomes a more specialised cell type.

One aspect of the invention provides an in vitro method for deriving embryonic stem cells.

The expression "a molecule capable of stimulating the activity of the endogenous E-cadherin" is to be understood as a molecule capable of recognising the endogenous E-cadherin, so that the adhesion complex comprising the E-cadherin-β-catenin is modified. In a preferred embodiment the molecule capable of stimulating the activity of the endogenous E-cadherin is an exogenous E-cadherin. In another preferred embodiment, the molecule capable of stimulating the activity of the endogenous E-cadherin is a chimeric E-cadherin. In a still more preferred embodiment, the chimeric E-cadherin is E-2153 (Sigma), MDL number MFCD03456676, which consists of the extracellular domain of mouse E-Cadherin (amino acids 1-709) fused to the carboxy-terminal 6X histidine tagged Fc region of human IgG1 expressed in mouse NSO cells. Other examples without limitation of molecules capable of stimulating the activity of the endogenous E-cadherin are antibodies, and more particularly monoclonal ones.

Guidance as to how to determine the appropriate incubation time of the above method to induce the division of the blastomere but not the differentiation of the blastomere can be derived following the examples disclosed. It is evident that the values given there serve as a first reference. The person skilled in the art is able to know how to determine the incubation times to be used in the method of the underlying invention. In a preferred embodiment the appropriate time is between (2-72) hours.

The blastomere obtained from the embryo is either co-cultured with a suitable cell to produce an ESC or cultured in conditioned medium, i.e. a medium where previously ESC have been grown. Any procedure known in the art can be used in the method of the underlying invention. Such procedures include but are not limited to co-culturing the blastomeres with feeder cells of mouse origin or human fibroblasts, in particular human foreskin fibroblasts.

The method described here has numerous important uses that will advance the field of stem cells research and developmental biology. ESC, ESC lines, TS cells and cells differentiated there can be used therapeutically in the treatment of numerous diseases and conditions. Additionally, these cells can be used in screening assays to identify factors that can be used to modulate the growth, differentiation, survival, or migration of these cells. The fact that ESC has the capacity to differentiate into a large number of tissues makes them especially interesting for the design of new therapies in general and of regenerative therapies in particular. As shown in the examples, the ESC could also be induced to differentiate to produce one or more mesodermal, endodermal or ectodermal cell type. Exemplary cell types include without limitation, hematopoietic SC, pancreatic beta cells, skin cells, vascular cells, etc. In summary, ESC themselves or cells differentiated thereof can be used therapeutically.

Also provided is an in vitro method inducing the proliferating of a single isolated blastomere obtained from an embryo before compaction, comprising the step of incubating the isolated blastomere, with a molecule capable of stimulating the activity of the endogenous E-cadherin during the appropriate time to induce the division of the blastomere but not the differentiation of the blastomere.

The above method of the present invention is useful as previous step in preimplantation genetic diagnosis to increase the number of blastomeres, and, hence, the genetic material available to carry out the genetic diagnostic test.

The methods of the present invention do not include any further use for commercial or industrial purpose than those above mentioned.

As it is shown in the examples, the efficiency of the ESC derivation process, as well as the blastomere division rate following the methods of the present invention are high (see also Table 1 of the examples).

The percentage of blastomere division after 24 hours of culture with the chimeric E-cad-Fc is 88.8%, a value significantly higher (p<0.0001) than that of the control group cultured without E-cad-Fc (44.6%), and also higher than that of the 1/8 blastomere group exposed to E-cad-Fc after 48 hours (67%).

Regarding the derivation efficiency, only 1 ESC line (1%) is obtained from 1/8 blastomeres exposed to E-cad-Fc for more than 24 hours. This derivation efficiency is similar to that of the control group without E-cad-Fc in the culture medium, in which only 5 ESC lines (2.2%) are derived. On the contrary, 42 ESC lines (33.6%) are derived when the E-cad-Fc is completely withdrawn after 24 hours. This ESC derivation rate is significantly higher (p<0.0001) than that of the group of the 1/8 blastomeres after a long exposure to E-cad-Fc and also higher than that of the control group.

Finally, the present invention also encompasses an in vitro method for deriving trophoblast stem cells comprising the step of incubating a single isolated blastomere obtained from an embryo before compaction, in a trophoblast stem cell medium and in the presence of a molecule capable of stimulating the activity of the endogenous E-cadherin during the appropriate time to induce the differentiation of the trophoblast.

The blastomere obtained from the embryo can be co-cultured with any suitable cell to produce a TSC. Any procedure known in the art can be used in the methods of the underlying invention. Such procedures include but are not limited to co-culture the blastomeres with feeder cells of mouse origin, or human fibroblasts, in particular human foreskin fibroblasts.

These TSC can be used for screening drugs which have said cells as therapeutic target. Another possible use of TSC is to regenerate or repare damaged placentas.

As it is shown in the examples, the efficiency of the TSC derivation process following the method of the present invention is high (see also Table 2 of the examples).

The blastomere isolated from an embryo, could be isolated using any suitable technique known in the art, with the proviso that the remaining embryo was not destroyed following obtaining the blastomere, in other words, the embryo remained viable. Exemplary techniques that could be used, without limitation, are chemical drilling using an acid Tyroid, and non-contact laser pulse described in detail in Material and Methods section of Veiga *et al.* (1994), and, Goosens *et al.* (2008), respectively. Both publications are incorporated by reference.

The blastomere could be isolated from a two-, four-, six- or eight-cell stage embryo. In a preferred embodiment, the blastomere could be isolated from an eight cell stage one.

The embryo can be any of any animals. In certain embodiments the embryo is a mammalian embryo. In other embodiments the mammalian embryo is a human embryo. Exemplary mammals include, but are not limited to, mice, rats, rabbits, dogs, cows, sheep, hamsters, pigs, and non-human primates.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### Embryo collection and blastomere isolation

Embryos were collected from female 129/Sv mice mated with male C57BI/6 mice were used. Prior to mating, females were subjected to a superovulation process by intraperitoneal injection of 5 international units (iu) of pregnant mare serum gonadotrophin (PMSG) followed by 5 iu of human chorionic gonadotrophin (hCG) 48 h after the first injection. Embryos at the 2-cell stage were obtained 48 hours after the hCG injection by flushing the oviducts with Hepes-buffered KSOM and were cultured in KSOM at 37°C under 5% CO2 until 8-cell and blastocyst stages were reached.

Groups of 1 or 2 blastomeres from 8-cell stage embryos (1/8 and 2/8 groups, respectively) were isolated by micromanipulation in a PBS solution without Ca⁺² and Mg⁺². The zona pellucida was disrupted with a 10 µm drilling micropipette containing Tyrode's acid solution and individual blastomeres were aspirated with a 20 µm diameter micropipette (Handyside *et al.,* 1989). The isolation technique used did not involve the destruction of the remaining embryos.

### Effect of the E-cadherin on the division capacity of single isolated blastomeres

1/8 blastomeres (n=125) were cultured in ESC culture conditions in the presence of 1.5 µg/ml of a chimeric E-cad-Fc consisting in the extracellular domain of mouse E-cadherin (amino acids 1-709) and the human IgG1 Fc domain. Said chimeric protein was used to allow distinguishing it from the endogenous mouse E-cad by using antibodies against the Fc human IgG1 domain. The ESC culture conditions consisted of a feeder cell monolayer cultured in 50 drops contained in a 60 nm Petri dish with ESC derivation DMEM medium supplemented with 100 µM 2-ß-mercaptoethanol, 1 mM L-glutamine, 1X non essential aminoacids, 10³ units/ml leukaemia inhibitory factor, 20% Knockout Serum Replacement and 0.1 mg/ml adrenocorticotropic hormone (Wakayama *et al.,* 2007, González *et al.*, 2010). The feeder cell monolayer was obtained from STO mouse embryo fibroblasts (ECACC) inactivated by a treatment with mitomycin C during 3 hours at a concentration of 10 µg/ml in order to produce feeder cells. The feeder cells were plated using Dulbecco's modified Eagle's medium (DMEM) supplemented with 15% foetal calf serum (FCS).

The E-cad was actively removed 24 hours after blastomeres were seeded onto the feeder cell monolayer (short exposure) by culturing them in a Ca⁺² and Mg⁺² free medium.

As control groups, 1/8 blastomeres (n=222) and 2/8 blastomeres (n=30) were cultured in ESC derivation medium without E-cad-Fc.

### Effect of the E-cad on the ESC derivation capacity

To assess the effect of E-cad on the ESC derivation capacity, in addition to the culture of 1/8 blastomeres in ESC culture conditions in the presence of E-cad-Fc during 24 hours (as described above), 1/8 blastomeres (n=100) were cultured in ESC culture conditions in the presence of E-cad at a concentration of 1.5 µg to the culture medium before 1/8 blastomeres were seeded. After 48 hours, the E-cad was passively removed by diluting it through successive changes of fresh medium every 2 days (long exposure). As control groups, those of the previous experiment were used.

The efficiency of the ESC derivation and blastomere division following the detailed examples are summarised in Table 1 below:

| Group | n | % First division after 24h (n) | % ESC lines (n) |
|---|---|---|---|
| Control (without E-cad-Fc) | 222 | 44.6^{a} (99) | 2.2^{a} (5) |
| E-cad-Fc long exposure (>48h) | 100 | 67^{a} (67) | 1^{a} (1) |
| E-cad-Fc short exposure (24h) | 125 | 88.8^{b} (11) | 33.6^{b} (42) |

| | | | |
|---|---|---|---|
| ^{a,b} Values with different superscripts within the same column differ significantly | | | |

### Effect of E-cadherin on ICM and TSC proliferation

In all the above derivation experiments, structures resembling blastocysts (also known as pseudoblastocysts) were observed on the second day of culture. The mean number of ICM and TE in pseudoblastocysts derived from experiments with E-cad-Fc (n=25) and in control pseudoblastocysts (n=50) was estimated by inmunofluorescence (Oct4 for ICM and CDx2 for TE). In addition, 1/8 blastomeres (n=30) subjected to long exposure to E-cad-Fc were cultured in TSC derivation conditions. 1/8 blastomeres (n=25) and blastocysts (n=25) cultured in TSC conditions without E-cad-Fc were used as controls. The immunostaining protocol followed was the same used for stem cell colonies except that pseudoblastocysts were washed with block solution instead of PBS and that permeabilisation was performed overnight.

The efficiency of the TSC derivation following the detailed examples are summarised in Table 2 below:

**Table 2**

| Group | n | % TSC lines (n) |
|---|---|---|
| Control (1/8) | 25 | 4.0 (1) |
| E-cad-Fc long exposure (>48h) | 30 | 13.3(4) |

In the 1/8 blastomere group long exposed to E-cad-Fc (for more than 24 hours), 4 TSC lines (13.3%) were obtained. In the control 1/8 group cultured in TSC conditions and not exposed to E-cad-Fc, only 1 TSC line (4%) was derived. Moreover, the production of TSC lines from the long exposed group with E-cad-Fc (13.3%) was significantly different to their respective production of ESC lines (1 %).

### Characterisation of ESC and TSC

The pluripotency of the derived ESC lines was checked by subjected them to differentiating culture conditions to produce cells of the three germ layers: ectoderm, endoderm and mesoderm. First, ESC lines were subcultured to gelatine pre-coated dishes in the absence of feeder cells and LIF. Moreover, the KSR of the culture medium was replaced with FCS to favour differentiation. Culture medium was replaced every two days and after a period of 7 days, the lines were fixed.

The presence of the three layers was checked through the detection of the expression of the neuroepithelial stem cell protein (Nestin, ectodermal layer), alpha-fetoprotein (AFP, endodermal layer) and α-smooth muscle actin (α - SMA, mesodermal layer). Samples were examined with an epifluorescence microscope Olympus Bx60 and an image and analyzing system (Software Genus, 3.0 version).

Furthermore, the pluripotency was tested by immunofluoresce through the expression of pluripotency markers (Oct4, Sox2, and Nanog). Stem cell colonies were washed 3 times in PBS, fixed in 4% paraformaldehyde during 15 min and washed again 3 times in PBS. Blocking and permeabilisation was performed in PBS solution containing 0.5% Triton X-1 00, 3% goat serum and 0.2% sodium azide during 30 minutes. Incubation with primary antibodies was done overnight at 4°C. Then they were washed 3 times in PBS and incubated with the corresponding secondary antibody during 2 hours at room temperature. Samples were washed again in PBR and stained with Hoechst 33258 at 10 g/ml as a nuclear counterstaining.

### REFERENCES CITED IN THE APPLICATION

Chung et al. "Embryonic and extraembryonic stem cell lines derived from single mouse blastomeres", Nature, 2008, vol. 439, pp. 216-219.
Klimanskaya et al. "Human embryonic stem cell lines derived from single blastomeres", Nature, 2006, pp. 1-5.
Nagaoka et al.,"E-cadherin-coated plates maintain pluripotency ES cells without colony formation", PLos One 1, 2006, e15.
Mohamet et al. "Abrogation of E-.cadherin-mediated cellular aggregation allows proliferation of pluripotent mouse embryonic stem cells in shake flask bioreactors", PLoS One 5, 2010, e12921.
Veiga et al. "Twin pregnancy after preimplantation diagnosis for sex selection: case report", Human Reproduction, 1994, vol. 9, pp. 2156-2159.
Goosens et al. "Diagnostic efficiency, embryonic developmental and clinical outcome after the biopsy of one or two blastomeres for preimplantation genetic diagnosis", Human Reproduction, 2008, vol. 23, pp. 481-492.
Handyside et al. "Biopsy of human preimplantation embryos and sexing DNA amplification", Lancet, 1989, vol. 8634, pp. 347-349.
Wakayama et al., "Efficient establishment of mouse embryonic stem cells lines from single blastomeres and polar bodies", Stem Cells, 2007, vol. 4, pp. 986-993.
González et al., "Establishment of mouse embryonic stem cells from isolated blastomeres and whole embryos using three derivation methods", J. Assist. Reproduc. Genet., 2010, Doi:10.10007/s10815-010-9473-9.

## Claims

1. An in vitro method for deriving embryonic stem cells, the method comprising the steps of: (a) incubating a single isolated blastomere, obtained from an embryo before compaction, with a molecule capable of stimulating the activity of the endogenous E-cadherin during the appropriate time to induce the division of the blastomere but not the differentiation of the blastomere; and (b) incubating the blastomeres resulting from step (a) in an embryonic stem cell medium.

2. An in vitro method for inducing the proliferation of a single isolated blastomere obtained from an embryo before compaction, comprising the step of incubating the isolated blastomere, with a molecule capable of stimulating the activity of the endogenous E-cadherin during the appropriate time to induce the division of the blastomere but not the differentiation of the blastomere.

3. The method according to any of the previous claims, wherein the molecule capable of stimulating the activity of endogenous E-cadherin is exogenous E-cadherin.

4. The method according to any of the previous claims, wherein the molecule capable of stimulating the activity of endogenous E-cadherin is a chimeric E-cadherin.

5. The method according to claim 4, wherein the chimeric E-cadherin consists of the extracellular domain of mouse E-cadherin (amino acids 1-709) and the human IgG1 Fc domain.

6. The method according to any of the claims 1-2, wherein the molecule is an antibody.

7. The method according to any of the previous claims wherein the isolated embryo is selected from human and mouse.
